(19)

Europäisches
Patentamt
European
Patent Office
Office européen
des brevets

(11) **EP 4 471 113 A1**

(12) **EUROPEAN PATENT APPLICATION**
published in accordance with Art. 153(4) EPC

(43) Date of publication:
**04.12.2024 Bulletin 2024/49**

(21) Application number: **22924380.3**

(22) Date of filing: **14.12.2022**

(51) International Patent Classification (IPC):
*C11B 1/02* (2006.01)      *C11B 1/10* (2006.01)
*C11B 3/00* (2006.01)      *C12P 7/6463* (2022.01)

(52) Cooperative Patent Classification (CPC):
**C11B 1/02; C11B 1/10; C11B 3/00; C12P 7/6463**

(86) International application number:
**PCT/KR2022/020311**

(87) International publication number:
**WO 2023/146127 (03.08.2023 Gazette 2023/31)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.01.2022 KR 20220012556**

(71) Applicant: **CJ CheilJedang Corporation
Seoul 04560 (KR)**

(72) Inventors:
• **JEONG, A Young
  Seoul 04560 (KR)**
• **AN, Jungap
  Seoul 04560 (KR)**
• **LEE, In
  Seoul 04560 (KR)**

(74) Representative: **Reddie & Grose LLP
The White Chapel Building
10 Whitechapel High Street
London E1 8QS (GB)**

(54) **BIO-OIL EXTRACTION METHOD WITH IMPROVED OIL RECOVERY RATE BY USING COOLING PROCESS**

(57)      The present invention relates to a bio-oil extraction method with an improved oil recovery rate by using a cooling process. The bio-oil extraction method of the present invention, in which a process of cooling a suspension obtained by disrupting cells of a culture solution containing microalgae is further carried out, exhibits a high oil recovery rate, and does not comprise adding other additives to increase an oil recovery rate, thus enabling oil recovery and the use of remaining by-products as feed, whereby sustainable production is possible, and the recovered oil can be effectively used as a feed composition, a food composition, or the like.

EP 4 471 113 A1

**Description**

[TECHNICAL FIELD]

Cross-reference to related application(s)

The present application claims the benefit of the priority based on Korean Patent Application No. 10-2022-0012556 filed on January 27, 2022, and the entire contents disclosed in the documents of the corresponding Korean patent application are incorporated as a part of the present description.

The present invention relates to a method for extracting bio-oil using a cooling process, and through the method, bio-oil can be extracted with a high oil recovery rate from microalgae.

[BACKGROUND ART]

Docosahexaenoic acid (DHA), an omega-3 polyunsaturated fatty acid having several advantages in preventing cardiovascular diseases such as hypertension angina·myocardial infarction, is not generally synthesized in the human body, and it should be consumed by humans, so it is considered an essential fatty acid. This DHA is mainly contained in fish oil. In general, it is found in salmon, sardine, tuna and the like a lot, so it is extracted from the liver of fish, and raw materials extracted from the liver of cod have been used the most worldwide. However, such DHA oil production from fish oil causes several problems. Destruction of the marine ecosystem due to overfishing and the risk of exposure to harmful substances to the human body as organic pollutants such as mercury, heavy metals, dioxins, and the like which are marine environmental pollutants are contained in fish oil have been constantly raised.

DHA, an omega-3 extracted from microalgae solves problems of omega-3 DHA originated from fish oil. Microorganisms of the genus Thraustochytrium and genus Schizochytrium can produce polyunsaturated fatty acids. A preparation method of an omega-3 polyunsaturated fatty acid by microalgae has been known by Ellenbogen (Ellenbogen B. B et al, Comparative Biochemistry and Physiology, 29:805-811 (1969)). Such DHA oil production through microalgae adopts a method of culturing only pure microalgae under a 100% clean condition, so there is no risk of destroying the marine ecosystem. In addition, mass production according to the culturing method is possible, so it is possible to supply with stable quality. Furthermore, it has a higher unsaturated fatty acid content than fish oil, and has a low risk of extracting impurities such as heavy metals and the like, so it is in the spotlight, and its market is also increasing.

A general method for preparing oil comprising polyunsaturated fatty acids (PUFA) from microalgae goes through processes of fermenting and culturing microalgae comprising a target substance in a reactor, recovering and drying, and extracting using a solvent. However, extraction using an organic solvent requires explosion-proof equipment, and it causes an increase in the preparation cost of the extraction according to the solvent recovering process. In addition, there is a risk of residual solvents in oil. In order to solve this problem, there is a need for a method for extracting oil from cells without using organic solvents. In case of solventless extraction, as a method for obtaining oil without using or minimizing organic solvents such as hexane, a method such as enzymatic decomposition, heat treatment and the like is one of methods of solventless extraction. In case of solventless extraction, oil can be obtained by a method such as centrifugation or natural immersion using a difference in specific gravity between common oil and other polar substance, and there is an advantage of excluding use of organic solvents that may be harmful to the human body. However, there is a problem in that phospholipids derived from an outer wall of a microorganism that interferes with separation of oil from microalgae act as an emulsifier and reduce an oil recovery rate.

As a method to solve this problem, there is a method of adding acid and base chemicals for pH adjustment or injecting a large amount of salt material such as sodium chloride (US 16/473805). However, this method fails to feed by-products remaining after extracting oil and results in rapid corrosion of an equipment.

[Prior art]

[Patent document]

(Patent document 1) U.S. Patent Publication US 2019/0323043 A

[DISCLOSURE]

[TECHNICAL PROBLEM]

One embodiment of the present application
provides a method for extracting oil from a microalgae culture medium comprising; 1) obtaining a cell lysate from a microalgae culture medium;

    2) recovering supernatant by centrifuging the cell lysate;
    3) cooling the supernatant;
    4) heating the cooled supernatant; and
    5) recovering oil by centrifuging the heated supernatant.

[TECHNICAL SOLUTION]

Each description and embodiment disclosed in the present application can be applied also to each other description and embodiment. In other words, all combinations of various elements disclosed in the present application fall within the scope of the present application. In addition, the scope of the present application is not to be construed as being limited by specific descriptions described below. Furthermore, those skilled in the art can recognize or confirm many equivalents to specific aspects of the present application disclosed in the present application. Moreover, these equivalents are intended to be included in the present application.

One embodiment of the present application

provides a method for extracting oil from a microalgae culture medium comprising;

    1) obtaining a cell lysate from a microalgae culture medium;
    2) recovering supernatant by centrifuging the cell lysate;
    3) cooling the supernatant;
    4) heating the cooled supernatant; and
    5) recovering oil by centrifuging the heated supernatant.

The term used in the present description, "microalgae" means small algae that cannot be seen with naked eyes and can only be seen through a microscope, and various kinds are included in the microalgae, and it includes even strains that cannot photosynthesize and grow only as heterotrophs. Such microalgae can fix carbon dioxide and have high protein and lipid contents, and can be utilized as biomass for producing food, feed, or fuel. Specifically, the lipid component contained in the microalgae can be used as a raw material for production of biodiesel oil used as liquid fuel.

The present description, the microalgae may be a Schizochytrium sp. strain, a Thraustochytrium sp. strain, a Japonochytrium sp. strain, a Ulkenia sp. strain, a Crypthecodinium sp. strain, a Haliphthoros sp. strain, and more preferably, it may be a Schizochytrium sp. strain, or a Thraustochytrium sp. strain, and most preferably, it may be a Schizochytrium sp. strain.

The term used in the present description, "culture medium" includes products produced by culturing the microalgae, and it can be interchangeably used with the term "fermented solution", and specifically, it may be a culture medium comprising microalgae or a culture filtrate obtained by removing the microalgae from the culture medium, but not limited thereto.

The microalgae culture medium may be prepared by inoculating the microalgae into a microalgae culture medium according to a culture method known in the art.

The term used in the present description, "lysate" refers to a solution containing contents of lysed cells, and it can be obtained by lysing by treating an enzyme to the microalgae culture medium or lysing by a physical method.

The term used in the present description, "supernatant" may mean liquid located at the top of the centrifuged liquid, and may be interchangeably used with "supernatant fluid." In addition, in the present description, "supernatant" in the step of centrifuging a cell lysate lysed by treating an enzyme to a microalgae culture medium or by a physical method may be interchangeably used with "an emulsified layer" or "emulsion" or "lysed suspension." Furthermore, the supernatant in the step of centrifuging after progressing cooling and/or heat treatment after recovering the supernatant may comprise "oil."

In the present invention, in the step 1), the cell lysate may be lysed by treating an enzyme to a microalgae culture medium.

The enzyme used for lysing of the microalgae culture medium may be alcalase, protease, cellulase, pectinase, chitinase, lipase, beta-glucanase, xylanase, mannanase, amylase, or a combination thereof, and preferably, it may be alcalase, but not limited thereto.

In the present description, in the step 1), the cell lysate may be lysed by a physical method.

The "physical method" means a method of lysing microalgae using an equipment capable of destroying cell walls of microalgae (namely, crusher), and a bead mill, a French pressure, a homogenizer (Braun homogenizer) or a microfluidizer, and preferably, a bead mill or a homogenizer may be used, and more preferably, a bead mill may be used.

In the present description, the centrifuging of the step 2) may be performed at 3000g to 4600g, 3200g to 4400g, 3400g to 4200g, or 3600g to 4000g for 1 minute to 10 minutes, 3 minutes to 8 minutes, or 4 minutes to 6 minutes.

In the example of the present invention, as the result of comparing the oil recovery rate according to the presence or absence of a centrifuging process before cooling treatment, it was confirmed that the oil recovery rate was low in case of going through a temperature adjusting process of cooling treatment and heat treatment without a centrifuging process, and the oil recovery rate was significantly increased, in case of going through a process of cooling treatment and heat treatment of the supernatant recovered by centrifuging a microalgae culture medium.

In the present invention, the cooling of the step 3) may be performed at a temperature of 45°C or less, 40°C or less, 35°C or less, 30°C or less, 5°C to 45°C, 5°C to 40°C, or 5°C to 35°C.

The cooling of the step 3) may be performed for 30 minutes or more, and may be performed for 40 minutes or more, and may be performed for 50 minutes or more, and may be performed for 30 minutes to 4 hours, but not limited thereto.

The time for performing the cooling of the step 3) may vary depending on the method for obtaining a cell lysate of the step 1). Specifically, when the cell lysate of the step 1) is obtained by treating an enzyme, the cooling of the step 3) may be performed for 1 hour to 5 hours, and may be performed for 1 hour and 30 minutes to 4 hours and 30 minutes, and may be performed for 2 hours to 4 hours, and may be performed for 2 hours and 30 minutes to 3 hours and 30 minutes, but not limited thereto. When the cell lysate of the step 1) is obtained using a physical method, the cooling of the step 3) may be performed for 30 minutes to 3 hours and 30 minutes, and may be performed for 30 minutes to 3 hours, and may be performed for 30 minutes to 2 hours and 30 minutes, and may be performed for 30 minutes to 2 hours, and may be performed for 30 minutes to 1 hour and 30 minutes, but not limited thereto.

In the present invention, the heating of the step 4) may be performed at a temperature of 50°C or more, 55°C or more, 60°C or more, 50°C to 75°C, 50°C to 70°C, 50°C to 65°C, 55°C to 75°C, 55°C to 70°C, or 55°C to 65°C

The heating of the step 4) may be performed for 30 minutes or more, and may be performed for 40 minutes or more, and may be performed for 50 minutes or more, and may be performed for 30 minutes to 4 hours, but not limited thereto.

The method for extracting oil from a microalgae culture medium of the present invention may improve an oil recovery rate.

In the present description, "oil" is interchangeably used with "bio-oil," and means all kinds of oil comprised in the microalgae, and for example, it includes polyunsaturated fatty acids (for example, omega-3 unsaturated fatty acids and omega-6 unsaturated fatty acids), saturated fatty acids such as lauric acid, myristic acid, palmitic acid, stearic acid and arachidic acid, and phospholipid and steroid-based compounds.

In the present description, the term "oil recovery rate" means an amount of extractable oil when oil is extracted through an experiment, among the intracellular oil content, that is, the crude fat content.

When oil is extracted by the method for extracting oil from a microalgae culture medium of the present invention, the oil recovery rate may be 70% or more, 75% or more, 80% or more, 85% or more, or 90% or more based on the total weight of the crude fat in the microalgae.

In the example of the present invention, it has been confirmed that the oil recovery rate is significantly increased when cooling treatment is performed and then heat treatment is performed, after centrifugation of the microalgae cell lysate, and therefore, the method for extracting oil of the present invention comprising centrifuging the cell lysate obtained from a microalgae culture medium, cooling and heating increases the oil recovery rate and does not add other additives, so remaining by-products after recovering oil can be turned into feed, and thus it can be usefully used as a method capable of sustainable production.

[ADVANTAGEOUS EFFECTS]

The method for bio-oil extraction in which a process of cooling suspension in which cells of a culture medium comprising microalgae are lysed of the present invention shows a high oil recovery rate, and in order to increase the oil recovery rate, other additives are not added, so by-products remaining after recovering oil can be turned into feed, and the sustainable production is possible, and the recovered oil can be usefully used as a composition for feed or a composition for food, or the like.

[MODE FOR INVENTION]

Hereinafter, the present invention will be described in more detail by examples. However, these examples are intended to illustratively describe at least one specific embodiment, and the scope of the present invention is not limited to these examples.

**Example 1. Bio-oil extraction of cell lysate using enzyme treatment**

**Example 1-1. Acquirement of cell lysate in which cell walls are lysed by enzyme treatment**

Culturing a microalgal Schizochytrium sp. strain for 60 hours was carried out in a 5L fermenter by supplying glucose carbon sources of 35% based on the total culture medium.

Culturing for about 20 hours was performed in a 500 mL flask under the condition of 30°C, 150 rpm using a sterilized MJW02 medium on the purpose of seed culture.

The seed cultured flask was aliquoted and inoculated in a 5L incubator (fermenter), and culturing was carried out in the sterilized MJW02 medium under the culturing environment 30°C, 500 rpm, 1.5 vvm condition to obtain a culture medium.

The culture medium was heated to 60°C, and then alcalase (2.4FG, Novozymes) was added by 0.2%(w/w) based on the weight of the culture medium for stirring reaction at 60°C for 3 hours, and thereby, a cell lysate in which extracellular walls are lysed was prepared.

**Example 1-2. Comparison of oil recovery rate according to presence or absence of cooling process**

After centrifuging the cell lysate obtained in Example 1-1 at 3800 g for 5 minutes, the supernatant was recovered using a spuit or a pipette and the improvement of the oil recovery rate according to presence or absence of cooling treatment was confirmed therefrom.

Specifically, the microalgae culturing of Example 1-1 was treated under the process order and process condition of 1-A, 1-B, 1-C, 1-D, 1-E and 1-F shown in Table 1 below, respectively, and then the oil recovery rate was compared. The heating and cooling rates were adjusted to $\pm\,0.5$ °C/min, and after reaching the set temperature, heating and cooling were performed as much as the time under the process condition. The oil recovery rate was calculated using the following equation by measuring the ratio of the recovered oil to the fat weight in the added culture medium. The weight of the recovered oil was measured after recovering the supernatant utilizing a spuit or a pipette after the final centrifuging step.

$$\text{Oil recovery rate (\%)} = \text{recovered oil weight} / (\text{culture medium weight} * \text{solid\%}) \text{ X } 100$$

[Table 1]

| Case | | Process order | Process condition | Oil recovery rate (%) |
|---|---|---|---|---|
| 1-A | 1 | Enzyme reaction | 60°C, 3hr | 0 |
| | 2 | Centrifugation | 3800g. 5min | |
| 1-B | 1 | Enzyme reaction | 60°C, 3hr | 20 |
| | 2 | Centrifugation | 3800g. 5min | |
| | 3 | Heat treatment after recovering supernatant | 60°C, 1hr | |
| | 4 | Centrifugation | 3800g. 5min | |
| 1-C | 1 | Enzyme reaction | 60°C, 3hr | 22 |
| | 2 | Centrifugation | 3800g. 5min | |
| | 3 | Heat treatment after recovering supernatant | 75°C, 1hr | |
| | 4 | Centrifugation | 3800g. 5min | |
| 1-D | 1 | Enzyme reaction | 60°C, 3hr | 88 |
| | 2 | Centrifugation | 3800g. 5min | |
| | 3 | Cooling treatment after recovering supernatant | 30°C, 3hr | |
| | 4 | Heat treatment | 60°C, 1hr | |
| | 5 | Centrifugation | 3800g. 5min | |

(continued)

| Case | | Process order | Process condition | Oil recovery rate (%) |
|---|---|---|---|---|
| 1-E | 1 | Enzyme reaction | 60°C, 3hr | 88 |
| | 2 | Centrifugation | 3800g. 5min | |
| | 3 | Cooling treatment after recovering supernatant | 25°C, 3hr | |
| | 4 | Heat treatment | 60°C, 1hr | |
| | 5 | Centrifugation | 3800g. 5min | |
| 1-F | 1 | Enzyme reaction | 60°C, 3hr | 8 |
| | 2 | Centrifugation | 3800g. 5min | |
| | 3 | Cooling treatment after recovering supernatant | 30°C, 3hr | |
| | 4 | Centrifugation | 3800g. 5min | |

As a result, as shown in Table 1, when the supernatant was not heat treated or cooling treated (Case 1-A), the oil recovery rate was shown as 0%, and when it was centrifuged immediately after heat treatment at 60°C for about 1 hour (Case 1-B), the oil recovery rate was shown as 20%. When it was heat treated at a higher temperature, 75°C for 1 hour (Case 1-C), the oil recovery rate was 22%, thereby confirming that the oil recovery rate was improved.

On the other hand, when the supernatant was cooling treated at 30°C for 3 hours, and then heat treated at 60°C for 1 hour (Case 1-D), and the supernatant was cooling treated at 25°C for 3 hours and then heat treated at 60°C for 1 hour (Case 1-E), a high oil recovery rate of 88% was shown, and it was confirmed that the oil recovery rate was significantly increased when cooling treatment was performed and then heat treatment was performed again.

However, in case of Case 1-F which went through the cooling process without heat treatment, rather, the oil recovery rate was low as 8%. The reason for this is considered that the extraction yield is low because it is difficult to recover saturated fatty acids comprised in the oil extracted from microalgae which are solids at a room temperature by centrifugation.

**Example 1-3. Comparison of oil recovery rate according to presence or absence of centrifuging process before cooling treatment**

In order to compare the oil recovery rate according to presence of absence of the centrifuging process before cooling treatment of the microalgae cell lysate in which cells were lysed through enzyme treatment, the following experiment was performed.

Specifically, the microalgae cell lysate of Example 1-1 was treated under the process order and process condition of 2-A and 2-B shown in Table 1 below, respectively, and then the oil recovery rate was compared. The heating and cooling rates were adjusted to ± 0.5 °C/min, and after reaching the set temperature, heating and cooling were carried out. The oil recovery rate was measured by the same method as Example 1-2.

[Table 2]

| Case | | Process order | Process condition | Recovery rate (%) |
|---|---|---|---|---|
| 2-A | 1 | Enzyme reaction | 60°C, 3hr | 9 |
| | 2 | Cooling treatment after recovering supernatant | 20°C, 3hr | |
| | 3 | Heat treatment | 60°C, 3hr | |
| | 4 | Centrifugation | 3800g. 5min | |
| 2-B | 1 | Enzyme reaction | 60°C, 3hr | 92 |
| | 2 | Centrifugation | 3800g. 5min | |
| | 3 | Cooling treatment after recovering supernatant | 20°C, 3hr | |
| | 4 | Heat treatment | 60°C, 3hr | |
| | 5 | Centrifugation | 3800g. 5min | |

As a result, as shown in Table 2, the oil recovery rate when the enzyme-treated microalgae cell lysate went through the temperature adjusting process of cooling treatment and heat treatment without the centrifuging process was low as 9% (Case 2-A). On the other hand, it was confirmed that when the supernatant recovered by centrifuging went through the same temperature adjusting process as Case 2-A, the oil recovery rate was significantly increased as 92% (Case 2-B). Through this, it was confirmed that when the enzyme-treated microalgae cell lysate was used as it is without the centrifuging process, even though the temperature adjusting process was gone through, the oil recovery was difficult, but in case that the supernatant in which microalgae cell walls and oil were mixed was used through the centrifuging process, when the temperature adjusting process of cooling treatment and heat treatment was gone through, the oil recovery rate was noticeably increased.

**Example 2. Bio-oil extraction of cell lysate using physical treatment**

**Example 2-1. Acquirement of microalgae cell lysate of which cell walls are lysed by physical treatment**

Culturing a microalgal Schizochytrium sp. strain for 60 hours was carried out in a 5L fermenter by supplying glucose carbon sources of 35% based on the total culture medium.

Culturing for about 20 hours was performed in a 500 mL flask under the condition of 30°C, 150 rpm using a sterilized MJW02 medium on the purpose of seed culture.

The seed cultured flask was aliquoted and inoculated in a 5L incubator (fermenter), and culturing was carried out in the sterilized MJW02 medium under the culturing environment 30°C, 500 rpm, 1.5 vvm condition to obtain a culture medium.

For the microalgae culture medium in which culturing was completed, using a bead mill equipment (Dyno-mill ECM-MULTI LAB), those with a bead size of 0.8 mm were filled in about 65% of a reactor and it was maintained at 30°C, and 1 pass was carried out as the rotation rate was 3600 rpm and the injection rate of the culture medium was 0.15 kg/min to obtain a cell lysate in which the cell lysing rate was 90% or more and the biomass concentration was 100 g/L or more, and the pH was 7, and the temperature was 30°C.

**Example 2-2. Comparison of oil recovery rate according to presence or absence of cooling process**

Improvement of the oil recovery rate according to cooling treatment was confirmed using the microalgae cell lysate by physical treatment obtained in Example 2-1.

Specifically, using the microalgae cell lysate by physical treatment in Example 2-1, it was treated under the process order and process condition of 3-A, 3-B, 3-C, 3-D, 3-E and 3-F shown in Table 3 below, respectively, and then the oil recovery rate was compared. A 500ml double jacket was connected to a circulator (Julabo FP50-HE) to adjust the temperature, and a process solution was injected in the double jacket to progress an experiment. The heating and cooling rates were adjusted to ± 1 °C/min, and after reaching the set temperature, heating and cooling were carried out for the time as much as the process condition of the table below. The oil recovery rate was measured by the same method as Example 1-2.

[Table 3]

| Case | | Process order | Process condition | Recovery rate (%) |
|---|---|---|---|---|
| 3-A | 1 | Bead mill treatment | 30°C | 1.5 |
| | 2 | Centrifugation | 3800g. 5min | |
| 3-B | 1 | Bead mill treatment | 30°C | 33.7 |
| | 2 | Centrifugation | 3800g. 5min | |
| | 3 | Heat treatment after recovering supernatant | 60°C, 1hr | |
| | 4 | Centrifugation | 3800g. 5min | |
| 3-C | 1 | Bead mill treatment | 30°C | 32.3 |
| | 2 | Centrifugation | 3800g. 5min | |
| | 3 | Heat treatment after recovering supernatant | 75°C, 1hr | |
| | 4 | Centrifugation | 3800g. 5min | |

(continued)

| Case | | Process order | Process condition | Recovery rate (%) |
|---|---|---|---|---|
| 3-D | 1 | Bead mill treatment | 30°C | 76 |
| | 2 | Centrifugation | 3800g. 5min | |
| | 3 | Cooling treatment after recovering supernatant | 25°C, 1hr | |
| | 4 | Heat treatment | 60°C, 1hr | |
| | 5 | Centrifugation | 3800g. 5min | |
| 3-E | 1 | Bead mill treatment | 30°C | 75.8 |
| | 2 | Centrifugation | 3800g. 5min | |
| | 3 | Cooling treatment after recovering supernatant | 15°C, 1hr | |
| | 4 | Heat treatment | 60°C, 1hr | |
| | 5 | Centrifugation | 3800g. 5min | |
| 3-F | 1 | Bead mill treatment | 30°C | 78.4 |
| | 2 | Centrifugation | 3800g. 5min | |
| | 3 | Cooling treatment after recovering supernatant | 5°C, 1hr | |
| | 4 | Heat treatment | 60°C, 1hr | |
| | 5 | Centrifugation | 3800g. 5min | |

As a result, as shown in Table 3, in case of no heat treatment or cooling treatment for the microalgae cell lysate (Case 3-A), the oil recovery rate was low as 1.5%, and in case of heat treatment at 60°C for about 1 hour and right after that, centrifuging (Case 3-B), the oil recovery rate was shown as 33.7%. In case of heat treatment at a higher temperature, 75°C for 1 hour (Case 3-C), the oil recovery rate was 32.3%, and it was confirmed that the oil recovery rate was not improved.

On the other hand, it was confirmed that in case of heat treatment at 60°C for 1 hour, after cooling treatment at 25°C for 1 hour for the microalgae cell lysate (Case 3-D), and in case of heat treatment at 60°C for 1 hour, after cooling treatment at 15°C for 1 hour for the microalgae cell lysate (Case 3-E), high recovery rates of 76% and 75.8%, respectively, were shown, and even in case of cooling treatment at 5°C for 1 hour (Case 3-F), a high oil recovery rate of 78.4% was shown, and thereby, it was confirmed that the oil recovery rate was significantly increased when cooling treatment was carried out and then heat treatment was performed again for the microalgae cell lysate by physical treatment.

### Example 2-3. Comparison of oil recovery rate according to presence or absence of centrifuging process before cooling treatment

In order to compare the oil recovery rate according to presence or absence of the centrifuging process before cooling treatment of the microalgae cell lysate in which cells were lysed by physical treatment, the following experiment was performed.

Specifically, except for proceeding in 2 passes at a rotation rate of 2400 rpm, and an injection rate of the culture medium of 0.15 kg/min, by the same method as Example 2-1, a microalgae cell lysing process solution in which cell walls were lysed using physical treatment was obtained. The obtained cell lysing process solution was put in a 50 ml falcon, and the temperature was adjusted in Thermomix, and it was stirred at 600 rpm, and thereby, the experiment was conducted. Using the microalgae suspension in which cells were lysed by physical treatment before centrifuging, it was treated under the process order and process condition of 4-A and 4-B shown in Table 4 below, respectively, and then the oil recovery rate was compared. The heating and cooling rates were adjusted to ± 1 °C/min, and after reaching the set temperature, heating and cooling were carried out for the time as much as the process condition of the table below. The oil recovery rate was measured by the same method as Example 1-2.

[Table 4]

| Case | | Process order | Process condition | Recovery rate (%) |
|---|---|---|---|---|
| 4-A | 1 | Bead mill treatment | 30°C, 2400rpm | 48.3 |
| | 2 | Cooling treatment after recovering supernatant | 20°C, 3hr | |

(continued)

| Case | | Process order | Process condition | Recovery rate (%) |
|---|---|---|---|---|
| | 3 | Heat treatment | 60°C, 3hr | |
| | 4 | Centrifugation | 3800g. 5min | |
| 4-B | 1 | Bead mill treatment | 30°C, 2400rpm | 95.4 |
| | 2 | Centrifugation | 3800g. 5min | |
| | 3 | Cooling treatment after recovering supernatant | 20°C, 3hr | |
| | 4 | Heat treatment | 60°C, 3hr | |
| | 5 | Centrifugation | 3800g. 5min | |

As a result, as shown in Table 4, the oil recovery rate, in case of going through the temperature adjusting process of cooling treatment and heat treatment without the centrifuging process for the microalgae cell lysate in which bead mill treatment was went through, was low as 48.3% (Case 4-A). On the other hand, it was confirmed that when the supernatant in which extracellular wall substances and oil were mixed, which were recovered by centrifuging the microalgae cell lysate in which bead mill treatment was went through at 3800 g for 5 minutes, were under the cooling and heat treatment as same as Case 4-A, the oil recovery rate was increased to 95.4% (Case 4-B).

**Claims**

1.  A method for extracting oil from a microalgae culture medium comprising;

    1) obtaining a cell lysate from a microalgae culture medium;
    2) recovering supernatant by centrifuging the cell lysate;
    3) cooling the supernatant;
    4) heating the cooled supernatant; and
    5) recovering oil by centrifuging the heated supernatant.

2.  The method for extracting oil from a microalgae culture medium according to claim 1, wherein the microalgae of the step 1) is a Schizochytrium sp. strain.

3.  The method for extracting oil from a microalgae culture medium according to claim 1, wherein the cell lysate of the step 1) is lysed by enzyme treatment or physical treatment.

4.  The method for extracting oil from a microalgae culture medium according to claim 3, wherein the physical treatment is physically lysing the microalgae culture medium using a bead mill, French pressure, homogenizer or microfluidizer.

5.  The method for extracting oil from a microalgae culture medium according to claim 1, wherein the centrifuging of the step 2) is performed at 3000g to 4600g.

6.  The method for extracting oil from a microalgae culture medium according to claim 1, wherein the cooling of the step 3) is performed at a temperature of 35°C or less.

7.  The method for extracting oil from a microalgae culture medium according to claim 6, wherein the cooling of the step 3) is performed for 30 minutes to 4 hours.

8.  The method for extracting oil from a microalgae culture medium according to claim 1, wherein the heating of the step 4) is performed at a temperature of 55°C or more.

9.  The method for extracting oil from a microalgae culture medium according to claim 8, wherein the heating of the step 4) is performed for 30 minutes to 4 hours.

10. The method for extracting oil from a microalgae culture medium according to claim 1, wherein the method improves the oil recovery rate of the extracted oil.

11. The method for extracting oil from a microalgae culture medium according to claim 10, wherein the oil recovery rate is 75% or more.

| | |
|---|---|
| **INTERNATIONAL SEARCH REPORT** | International application No. |
| | **PCT/KR2022/020311** |

**A.    CLASSIFICATION OF SUBJECT MATTER**

**C11B 1/02**(2006.01)i; **C11B 1/10**(2006.01)i; **C11B 3/00**(2006.01)i; **C12P 7/6463**(2022.01)i

According to International Patent Classification (IPC) or to both national classification and IPC

**B.    FIELDS SEARCHED**

Minimum documentation searched (classification system followed by classification symbols)

C11B 1/02(2006.01); A23D 9/00(2006.01); C11B 1/00(2006.01); C11B 5/00(2006.01); C11C 1/00(2006.01); C11C 1/08(2006.01); C12N 1/06(2006.01); C12P 7/06(2006.01); C12P 7/64(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 바이오오일 (bio oil), 조류 (algae), 세포 (cell), 냉각 (cool), 가열 (heat)

**C.    DOCUMENTS CONSIDERED TO BE RELEVANT**

| Category* | Citation of document, with indication, where appropriate, of the relevant passages | Relevant to claim No. |
|---|---|---|
| Y | KR 10-1732062 B1 (KOREA RESEARCH INSTITUTE OF BIOSCIENCE AND BIOTECHNOLOGY) 02 May 2017 (2017-05-02)<br>    See paragraph [0034]; and claim 1. | 1-11 |
| Y | KR 10-2014-0087054 A (DSM IP ASSETS B.V.) 08 July 2014 (2014-07-08)<br>    See paragraphs [0057]-[0059]; and claim 13. | 1-11 |
| A | KR 10-2013-0071629 A (DAESANG CORPORATION) 01 July 2013 (2013-07-01)<br>    See entire document. | 1-11 |
| A | KR 10-2014-0101135 A (UNIVERSITY INDUSTRY FOUNDATION, YONSEI UNIVERSITY WONJU CAMPUS) 19 August 2014 (2014-08-19)<br>    See entire document. | 1-11 |
| A | WO 2011-150411 A1 (SOLAZYME, INC.) 01 December 2011 (2011-12-01)<br>    See entire document. | 1-11 |

☐ Further documents are listed in the continuation of Box C.        ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 March 2023** | **17 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT
Information on patent family members

International application No.

PCT/KR2022/020311

| Patent document cited in search report | | | Publication date (day/month/year) | Patent family member(s) | | | Publication date (day/month/year) |
|---|---|---|---|---|---|---|---|
| KR | 10-1732062 | B1 | 02 May 2017 | None | | | |
| KR | 10-2014-0087054 | A | 08 July 2014 | CN | 100282745 | C | 01 November 2006 |
| | | | | CN | 100447860 | A | 08 October 2003 |
| | | | | EP | 1178118 | A1 | 06 February 2002 |
| | | | | EP | 1305440 | A2 | 02 May 2003 |
| | | | | EP | 1305440 | B1 | 09 June 2010 |
| | | | | EP | 1305440 | B2 | 30 March 2022 |
| | | | | JP | 2004-504849 | A | 19 February 2004 |
| | | | | JP | 2012-019794 | A | 02 February 2012 |
| | | | | JP | 2014-138598 | A | 31 July 2014 |
| | | | | JP | 2016-208974 | A | 15 December 2016 |
| | | | | KR | 10-1447912 | B1 | 11 November 2014 |
| | | | | KR | 10-1515317 | B1 | 24 April 2015 |
| | | | | KR | 10-1648277 | B1 | 12 August 2016 |
| | | | | KR | 10-2003-0059086 | A | 07 July 2003 |
| | | | | KR | 10-2010-0019579 | A | 18 February 2010 |
| | | | | KR | 10-2011-0030661 | A | 23 March 2011 |
| | | | | KR | 10-2012-0135322 | A | 12 December 2012 |
| | | | | KR | 10-2013-0109249 | A | 07 October 2013 |
| | | | | US | 2004-0067574 | A1 | 08 April 2004 |
| | | | | US | 7431952 | B2 | 07 October 2008 |
| KR | 10-2013-0071629 | A | 01 July 2013 | KR | 10-1350245 | B1 | 16 January 2014 |
| KR | 10-2014-0101135 | A | 19 August 2014 | None | | | |
| WO | 2011-150411 | A1 | 01 December 2011 | CN | 103097540 | A | 08 May 2013 |
| | | | | CN | 103124499 | A | 29 May 2013 |
| | | | | CN | 103124499 | B | 28 September 2016 |
| | | | | CN | 106135460 | A | 23 November 2016 |
| | | | | CN | 106135460 | B | 24 July 2020 |
| | | | | CN | 107058116 | A | 18 August 2017 |
| | | | | EP | 2575486 | A1 | 10 April 2013 |
| | | | | EP | 2575486 | A4 | 23 March 2016 |
| | | | | EP | 2575486 | B1 | 01 September 2021 |
| | | | | EP | 2576800 | A2 | 10 April 2013 |
| | | | | EP | 2576800 | A4 | 13 July 2016 |
| | | | | EP | 2576800 | B1 | 30 January 2019 |
| | | | | JP | 2013-528383 | A | 11 July 2013 |
| | | | | JP | 2013-533735 | A | 29 August 2013 |
| | | | | JP | 2016-005484 | A | 14 January 2016 |
| | | | | JP | 2016-104024 | A | 09 June 2016 |
| | | | | JP | 2017-104132 | A | 15 June 2017 |
| | | | | JP | 2019-062919 | A | 25 April 2019 |
| | | | | JP | 5868962 | B2 | 24 February 2016 |
| | | | | JP | 5996527 | B2 | 21 September 2016 |
| | | | | JP | 6150911 | B2 | 21 June 2017 |
| | | | | JP | 6542283 | B2 | 10 July 2019 |
| | | | | KR | 10-1899938 | B1 | 19 September 2018 |
| | | | | KR | 10-1916421 | B1 | 08 November 2018 |
| | | | | KR | 10-2013-0085374 | A | 29 July 2013 |
| | | | | KR | 10-2013-0087410 | A | 06 August 2013 |
| | | | | KR | 10-2018-0122486 | A | 12 November 2018 |

Form PCT/ISA/210 (patent family annex) (July 2022)

**INTERNATIONAL SEARCH REPORT**
Information on patent family members

International application No.

**PCT/KR2022/020311**

| Patent document cited in search report | Publication date (day/month/year) | Patent family member(s) | | Publication date (day/month/year) |
|---|---|---|---|---|
| | | KR 10-2148462 | B1 | 26 August 2020 |
| | | US 10006034 | B2 | 26 June 2018 |
| | | US 2011-0293785 | A1 | 01 December 2011 |
| | | US 2011-0294174 | A1 | 01 December 2011 |
| | | US 2012-0324784 | A1 | 27 December 2012 |
| | | US 2013-0004646 | A1 | 03 January 2013 |
| | | US 2013-0096211 | A1 | 18 April 2013 |
| | | US 2013-0102039 | A1 | 25 April 2013 |
| | | US 2014-0315267 | A1 | 23 October 2014 |
| | | US 2016-0024538 | A1 | 28 January 2016 |
| | | US 2016-0186191 | A1 | 30 June 2016 |
| | | US 2018-0327758 | A1 | 15 November 2018 |
| | | US 8592188 | B2 | 26 November 2013 |
| | | US 8765424 | B2 | 01 July 2014 |
| | | US 9109239 | B2 | 18 August 2015 |
| | | US 9255282 | B2 | 09 February 2016 |
| | | US 9279136 | B2 | 08 March 2016 |
| | | US 9657299 | B2 | 23 May 2017 |
| | | WO 2011-150410 | A2 | 01 December 2011 |
| | | WO 2011-150410 | A3 | 19 April 2012 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220012556
- US 16473805 B

- US 20190323043 A

**Non-patent literature cited in the description**

- **ELLENBOGEN B. B et al.** *Comparative Biochemistry and Physiology*, 1969, vol. 29, 805-811